(19) Europäisches Patentamt
European Patent Office
Office européen des brevets

(11) EP 4 803 502 A1

(12) EUROPEAN PATENT APPLICATION
published in accordance with Art. 153(4) EPC

(43) Date of publication:
09.09.2026 Bulletin 2026/37

(21) Application number: 24885746.8

(22) Date of filing: 30.10.2024

(51) International Patent Classification (IPC):
*C07C 68/04* (2006.01) *B01J 23/10* (2006.01)
*C07B 61/00* (2006.01) *C07C 69/96* (2006.01)

(52) Cooperative Patent Classification (CPC):
**B01J 23/10; C07B 61/00; C07C 68/04; C07C 69/96;** Y02P 20/141

(86) International application number:
PCT/JP2024/038655

(87) International publication number:
WO 2025/094979 (08.05.2025 Gazette 2025/19)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 31.10.2023 JP 2023187023

(71) Applicants:
- MITSUBISHI GAS CHEMICAL COMPANY, INC.
Chiyoda-ku
Tokyo 100-8324 (JP)
- N.E. Chemcat Corporation
Tokyo 1055127 (JP)

(72) Inventors:
- SHINKAI, Yousuke
Tokyo 125-8601 (JP)
- NAKAO, Kaiki
Tokyo 125-8601 (JP)
- HARADA, Hidefumi
Tokyo 125-8601 (JP)
- ISOBE, Takehiko
Tokyo 125-8601 (JP)
- UMEZU, Ryotaro
Tokyo 100-8324 (JP)
- IMADA, Shin-ichiro
Tokyo 100-8324 (JP)
- YAGISHITA, Ryo
Tokyo 125-8601 (JP)
- TAKAHASHI, Yoshinori
Tokyo 105-5127 (JP)
- UEMURA, Kazuma
Tokyo 105-5127 (JP)
- HANDA, Hyuga
Tokyo 105-5127 (JP)

(74) Representative: Hoffmann Eitle
Patent- und Rechtsanwälte PartmbB
Arabellastraße 30
81925 München (DE)

# (54) METHOD FOR PRODUCING CARBONATE ESTER, AND CATALYTIC STRUCTURE FOR PRODUCING CARBONATE ESTER

(57) It cannot be said that the reaction rate of a carbonate ester production reaction based on carbon dioxide and a monohydric alcohol has been sufficiently improved by conventional catalysts, and higher reaction efficiency is required. In addition, conventional catalyst-supporting structures used in carbonate ester production reactions cannot be said to have always had sufficiently strong structures. The above problems have been solved by a method for producing a carbonate ester. Specifically, the method for producing a carbonate ester comprises a step for making a monohydric alcohol and carbon dioxide react in the presence of a catalyst structure to produce a carbonate ester, wherein the catalyst structure includes a substrate and a catalyst layer formed on at least a portion of the surface of the substrate and containing at least a solid catalyst and a binder, the solid catalyst contains particulate cerium oxide, and the binder contains cerium oxide as a uniform component.

EP 4 803 502 A1

Figure 1

100
80
72
60
73
P
G
74
P
G
82
84
76
78
86

## Description

### TECHNICAL FIELD

The present invention relates to a method for producing a carbonate ester and to a catalytic structure for producing a carbonate ester.

### BACKGROUND ART

Interest in global warming has increased in recent years. The COP (Conference of the Parties), which discusses international frameworks for reducing greenhouse gas emissions, etc., has set the goal of limiting the peak of greenhouse gas emissions as early as possible and reducing it rapidly according to the latest scientific findings. The COP21 Paris Agreement states that all countries should strive to design and submit a long-term low greenhouse gas emission development strategy, and Japan has designed a long-term goal of 80% greenhouse gas emission reduction by 2050.

Among the anthropogenic greenhouse gases, carbon dioxide is estimated to have the largest impact and so the development of countermeasure techniques for reducing carbon dioxide emissions is being enthusiastically pursued in various fields. As one of the countermeasure techniques, several attempts have been proposed to convert emitted carbon dioxide into useful substances. However, since a large amount of energy is required to convert carbon dioxide into another substance, development of an effective catalyst for accelerating the reaction is desired. Additionally, in order to achieve a technique that contributes to the reduction of carbon dioxide, it is necessary to produce a useful and commercially desired substance.

Meanwhile, carbonate esters are very useful compounds that can be used as an additive such as a gasoline additive to improve the octane number, or a diesel fuel additive to reduce particles in exhaust gas, also as an alkylation agent, a carbonylation agent, a solvent or the like that are used to synthesize a resin or an organic compound such as a polycarbonate, a urethane, a pharmaceutical, or an agrochemical, or as an electrolyte for lithium batteries, a raw material of a lubricant, or a raw material of an oxygen absorber for preventing rust on boiler pipes.

A carbonate ester is a generic term for a compound in which one or two of the two hydrogen atoms in carbonic acid $CO(OH)_2$ are replaced by an alkyl or aryl group, and which has the structure RO-C(=O)-OR' (where R and R' each represent a saturated hydrocarbon group or an unsaturated hydrocarbon group). Therefore, if such a compound can be efficiently produced from carbon dioxide, which is a compound equivalent to carbonic acid, this could be a useful measure for reducing carbon dioxide.

When a carbonate ester is synthesized directly from carbon dioxide and an alcohol, the reaction is known to proceed dramatically faster in the presence of a solid catalyst and a nitrile hydration agent (see, for example, Patent literature 1). There is also a known example where a catalyst is immobilized on a catalyst-supporting structure and used in a solution reaction system (see, for example, Patent literature 2).

### PRIOR ART DOCUMENTS

### PATENT DOCUMENTS

Patent literature 1: Japanese Patent Publication No. 2012-162523
Patent literature 2: WO2020/013135

### SUMMARY OF THE INVENTION

### PROBLEMS TO BE SOLVED BY THE INVENTION

Conventional catalysts do not sufficiently improve the reaction rate of the carbonate ester generation reaction between carbon dioxide and a monohydric alcohol, and thus higher reaction efficiency is required. Additionally, the conventional catalyst-supporting structures used for the carbonate ester generation reactions have not always been robust enough.

### MEANS FOR SOLVING THE PROBLEMS

In the course of the diligent study to solve the above-mentioned problems, the present inventors have found that if a binder having a predetermined component is used with a catalyst, the efficiency of the carbonate ester generation

reaction can be sufficiently improved and a catalyst-supporting structure with a robust structure can be realized. The present invention includes a method for producing a carbonate ester, and the like as follows.

[1] A method for producing a carbonate ester, comprising:

a step of reacting a monohydric alcohol and carbon dioxide in the presence of a catalytic structure to produce a carbonate ester,
wherein the catalytic structure comprises a substrate, and a catalyst layer formed on at least a portion of the surface of the substrate and containing at least a solid catalyst and a binder,
the solid catalyst comprises particulate cerium oxide, and
the binder comprises cerium oxide as a homogeneous component.

[2] The method for producing a carbonate ester according to [1] above, wherein the cerium oxide contained in the binder is derived from at least one of the following cerium compounds: cerium acetate, cerium sulfate, and cerium nitrate.
[3] The method for producing a carbonate ester according to [2] above, further comprising a binder generating step for generating the binder,
wherein, in the binder generating step, the cerium compound is calcined to obtain cerium oxide.
[4] The method for producing a carbonate ester according to any of [1]-[3] above, wherein the content of the cerium oxide contained as the binder in the catalyst layer is adjusted to fall within a predetermined range.
[4a] The method for producing a carbonate ester according to [2] or [3] above, e.g. [2] above, wherein the content of the cerium oxide derived from the cerium compound in the catalyst layer is 1.0-10% by weight relative to the total weight of the catalyst layer.
[4b] The method for producing a carbonate ester according to any one of [1]-[3] above, e.g. [1] above, wherein the content of the cerium oxide as a homogeneous component in the catalyst layer is 1.0-10% by weight relative to the total weight of the catalyst layer.
[5] The method for producing a carbonate ester according to any one of [1]-[4] above, e.g., [1] above, wherein the substrate is ceramic.
[6] The method for producing a carbonate ester according to any one of [1]-[5] above, e.g. [1] above, wherein the mass of the supported solid catalyst in the catalyst layer is 15 $g/m^2$ or more but 200 $g/m^2$ or less.
[7] The method for producing a carbonate ester according to any one of [1]-[6] above, e.g., [1] above, wherein a hydration agent is used to remove by-produced water in the carbonate ester generation reaction.
[8] A catalytic structure for producing a carbonate ester, comprising:

a substrate; and
a catalyst layer formed on at least a portion of the surface of the substrate and containing at least a solid catalyst and a binder,
wherein the solid catalyst comprises particulate cerium oxide, and
the binder comprises cerium oxide as a homogeneous component.

[9] The catalytic structure for producing a carbonate ester according to [8] above, wherein the content of the cerium oxide as a homogeneous component in the catalyst layer is 1.0-10% by weight relative to the total weight of the binder.
[10] The catalytic structure for producing a carbonate ester according to [8] or [9] above, e.g., [8] above, wherein the substrate is ceramic.
[11] The catalytic structure for producing a carbonate ester according to any one of [8]-[10] above, e.g. [8] above, wherein the mass of the supported solid catalyst in the catalyst layer is 15 $g/m^2$ or more but 200 $g/m^2$ or less.
[12] The catalytic structure for producing a carbonate ester according to any one of [8]-[11] above, e.g., [8] above, wherein the substrate has a honeycomb structure and the cell density of the substrate is 15-200 ($cells/cm^2$), or the wall thickness of the substrate is 0.01-2.0 mm.
[12a] The catalytic structure for producing a carbonate ester according to [12] above, wherein the cell density of the substrate is 15-200 ($cells/cm^2$), and the wall thickness of the substrate is 0.01-2.0 mm.

## ADVANTAGEOUS EFFECT OF THE INVENTION

According to the present invention, the reaction rate of the carbonate ester generation reaction can be enhanced and a carbonate ester can be produced in an efficient manner. In addition, a catalytic structure for producing a carbonate ester of the present invention has a robust structure, which can prevent a catalyst from powdering and desorbing while

maintaining excellent catalytic efficiency, even after long-term use.

BRIEF DESCRIPTION OF DRAWINGS

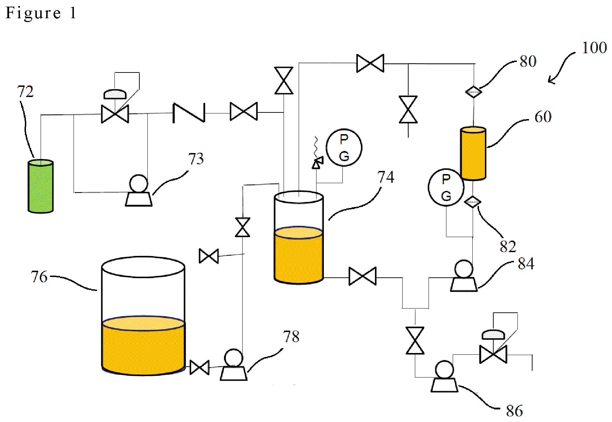

[Figure 1] A schematic diagram showing a specific example of a carbonate ester production facility, including various devices such as a carbonate ester production device having a catalytic structure.

DESCRIPTION OF EMBODIMENTS

Hereinafter, a preferred embodiment of the present invention will be described in detail.

[1. Catalytic structure for producing carbonate ester]

First, a preferred embodiment of a catalytic structure for producing a carbonate ester of the present invention will be described. The catalytic structure for producing a carbonate ester (hereinafter also simply referred to as a "catalytic structure") catalyzes generation of a carbonate ester from carbon dioxide and a monohydric alcohol, preferably in the presence of a hydration agent.

The catalytic structure for producing a carbonate ester according to the present embodiment has a substrate, and a catalyst layer formed on at least a portion of the surface of the substrate and containing at least a solid catalyst and a binder, The solid catalyst in the catalyst layer contains at least particulate cerium oxide. Furthermore, the binder in the catalyst layer contains cerium oxide as a homogeneous component. In this embodiment, the catalytic structure for producing a carbonate ester is provided with an intermediate layer composed of an inorganic binder sandwiched between the catalyst layer and the substrate.

Hereinafter, each component of the catalytic structure for producing a carbonate ester will be described.

(1.1. Substrate)

The substrate is a catalyst-supporting structure for supporting a solid catalyst. By supporting the solid catalyst on the surface of the substrate, uneven distribution of the solid catalyst in a reaction vessel during the reaction can be suppressed and local temperature variations in the reaction vessel due to the generated reaction heat can be reduced compared to the case where a powdery catalyst is used as is. Therefore, according to this embodiment, a carbonate ester as the resulting product can be produced with high efficiency.

Furthermore, even if the activity of the solid catalyst decreases as the reaction is continued for a long period of time, it can be removed from the reaction vessel and then regenerated by a heat treatment or the like, thereby restoring its function and facilitating continuous use over an extended period of time. As will be described in detail below, the component of a solid catalyst 40 can be adjusted to allow the catalyst to maintain good catalytic function for a longer period of time, thus reducing the frequency of the regeneration treatment.

The substrate is not particularly limited and may be of any material, shape, and dimensions as long as it allows formation of a catalyst layer containing a solid catalyst. In particular, the substrate preferably has communication holes that serve as a distribution pathway for raw material compounds such as a monohydric alcohol, carbon dioxide, etc. The presence of such communication holes in the substrate improves the diffusion efficiency of the monohydric alcohol and carbon dioxide as raw materials and facilitates the recovery of products such as a carbonate ester and by-produced water. Furthermore, formation of the catalyst layer on the surface of the communication holes increases the contact area between the catalyst layer and the monohydric alcohol and carbon dioxide as raw materials, thereby improving the efficiency of the reaction for generating a carbonate ester.

The shape of the substrate may be, for example, porous (e.g., foam-like), corrugated, honeycomb-like (monolithic), mesh-like, columnar, cylindrical, or the like. The substrate is preferably porous, foam-like, honeycomb-like, or mesh-like. These shapes are provided with communication holes, which allows them to achieve the advantage of having communication holes as described above. In particular, when the substrate has a honeycomb-like shape, the specific surface area of the communication holes can be made relatively large, while maintaining excellent physical strength and shape stability of the substrate.

The material that makes up the substrate is preferably, but is not particularly limited to, a ceramic material such as cordierite, mullite, silicon carbide, alumina, silica, titania, zirconia, or ceria, or a metallic material such as a stainless steel or an aluminum steel. These materials may also be used in combination.

Among the aforementioned substrate material, ceramic and metallic materials are preferable. Accordingly, the substrate is preferably a ceramic honeycomb or a metal honeycomb, and particularly preferably a ceramic honeycomb.

If the substrate is formed of a ceramic, the adhesion of the catalyst layer to the substrate can be further improved. Meanwhile, the thermal conductivity of the substrate can be improved by using a metal, such as a stainless steel (a

martensitic, ferritic, austenitic, austenitic-ferritic duplex, or precipitation hardening stainless steel) as the material of the substrate.

When a honeycomb structure or the like, which has cells, is used as the substrate, the thickness of the cell wall is, for example, 0.01-2.0 mm (about 0.4-80 mil), preferably 0.1-1.5 mm (about 4-60 mil), and more preferably 0.5-1.3 mm (about 2-50 mil). While the range mentioned here presents the thickness of the cell wall of the substrate, the thickness of the cell wall of a catalyst-supporting structure, in which a catalyst layer is layered on the surface of the substrate, preferably falls in a range whose upper and lower limits are obtained by adding the thickness of the catalyst layer described below to the aforementioned upper and lower limits, respectively.

In addition to the material, shape, and cell wall thickness described above, cell density is also a factor that determines the structure of the substrate having cells. Cell density is expressed as the number of cells per unit area of the cross-section perpendicular to the axis line of the honeycomb structure. The cell density of the substrate, which preferably has a honeycomb-like shape, is 15-200 cells/cm$^2$ (about 200-1,300 cells/inch$^2$), preferably 20-150 cells/cm$^2$ (about 130-970 cells/inch$^2$), more preferably 25-120 cells/cm$^2$ (about 160-770 cells/inch$^2$) or 30-100 cells/cm$^2$ (about 193-645 cells/inch$^2$), and particularly preferably 45-93 cells/cm$^2$ (about 290-600 cells/inch$^2$) or 62-93 cells/cm$^2$ (about 400-600 cells/inch$^2$).

(1.2. Catalyst layer)

The catalyst layer is formed on at least a portion of the surface of the substrate. The catalyst layer has a solid catalyst containing at least particulate cerium oxide, which catalyzes the reaction that generates a carbonate ester from carbon dioxide and a monohydric alcohol. Such a catalyst layer is preferably formed on the inner wall surface of the communication holes in the substrate. More preferably, the catalyst layer covers the entire surface with which the raw material substrates for the carbonate ester formation reaction, including carbon dioxide, a monohydric alcohol, etc., make contact, such as the inner wall surface of the communication holes.

The catalyst layer contains at least a solid catalyst and a binder. Cerium oxide, an essential component of the solid catalyst, has excellent catalytic activity in the reaction that generates a carbonate ester from carbon dioxide and a monohydric alcohol. On the other hand, cerium oxide generally tends to powderize, but in this embodiment, cerium oxide is firmly immobilized on the substrate by a binder that will be described below, and thus desorption is suppressed and powdering can be prevented.

The catalyst may contain one or more catalysts other than cerium oxide. Such a catalyst may be any catalyst that catalyzes the reaction between carbon dioxide and a monohydric alcohol, and examples thereof include a tin compound, a thallium compound, a nickel compound, a vanadium compound, a copper compound, an alkali carbonate, zirconium oxide, titanium oxide, and a rare earth element other than cerium (especially an oxide thereof). Among these, zirconium oxide is preferred because of its high catalytic activity. In this case, the proportion of cerium oxide in the solid catalyst is, for example, 5 at.% or more, and preferably 20 at.% or more. The proportion of cerium oxide in the solid catalyst may also be 100 at.%.

When a cerium oxide catalyst is used continuously in a direct synthesis reaction of a carbonate ester, its catalytic activity decreases over time, requiring frequent catalyst regeneration treatments. However, high catalytic activity can be maintained by adjusting the catalyst components. For example, when a rare earth element other than cerium is added as an auxiliary catalyst component, the catalytic activity of the solid catalyst tends to remain high for a long period of time during the carbonate ester generation reaction.

Examples of the rare earth element other than cerium include scandium, yttrium, lanthanum, praseodymium, neodymium, promethium, samarium, europium, gadolinium, terbium, dysprosium, holmium, erbium, thulium, ytterbium, and lutetium. Among these rare earth elements, it is preferable to add lanthanum, praseodymium, and gadolinium to the solid catalyst, and particularly preferable to use lanthanum. These metal elements are mainly contained in the solid catalyst as oxides or are present in the surface layer of the solid catalyst.

The proportion of an auxiliary catalyst component (e.g. an oxide of a rare earth element) in the solid catalyst is preferably 0.01-10 mass%, more preferably 0.05-5.0 mass%, still more preferably 0.1-2.5 mass%, and particularly preferably 0.2-2.0 mass%, for example, 1 mass%, relative to the total mass of the solid catalyst.

The amount of the supported solid catalyst in the catalyst layer is preferably 15 g/m$^2$ or more but 200 g/m$^2$ or less, more preferably 20 g/m$^2$ or more but 150 g/m$^2$ or less, and particularly preferably 25 g/m$^2$ or more but 100 g/m$^2$ or less, or 25 g/m$^2$ or more but 80 g/m$^2$ or less, relative to the unit area of the catalyst layer. In a catalytic structure containing such a solid catalyst, the reaction efficiency of the catalytic reaction can be enhanced.

If the amount of the supported solid catalyst is less than 15 g/m$^2$, the reaction efficiency of the catalytic reaction by the catalytic structure may not be sufficient. On the other hand, if the amount of the supported solid catalyst exceeds 200 g/m$^2$, diffusion of the raw material substrate into the deeper part of the catalyst layer may become an obstacle and actually reduce the reaction efficiency.

Accordingly, there is no simple positive correlation between the amount of the supported solid catalyst and the

reaction efficiency, and there may be a range for suitable amount of the supported solid catalyst to achieve high reaction efficiency. A catalytic structure that supports the solid catalyst within the aforementioned content range can maintain high reactivity, even at a high feed rate, also allowing the reactor to be relatively small. The cost of a plant including such a catalytic structure can be kept low.

From the viewpoint of the reaction efficiency of the catalytic reaction using the catalytic structure, and the like, the amount of the supported solid catalyst in the catalyst layer is, for example, 10 $g/m^2$ or more but 200 $g/m^2$ or less, preferably 15 $g/m^2$ or more but 150 $g/m^2$ or less, more preferably 15 $g/m^2$ or more but 70 $g/m^2$ or less, and still more preferably 15 $g/m^2$ or more but 30 $g/m^2$ or less.

Similarly, from the viewpoint of the reaction efficiency of the catalytic reaction using the catalytic structure, and the like, the amount of the supported cerium oxide in the catalyst layer is, for example, 10 $g/m^2$ or more but 200 $g/m^2$ or less, 15 $g/m^2$ or more but 200 $g/m^2$ or less, preferably 15 $g/m^2$ or more but 150 $g/m^2$ or less, more preferably 15 $g/m^2$ or more but 70 $g/m^2$ or less, and still more preferably 15 $g/m^2$ or more but 30 $g/m^2$ or less.

The thickness of the catalyst layer is, for example, 12 μm-150 μm. The thickness of the catalyst layer in conventional products is generally above 5 μm or less, whereas the catalyst layer included in the catalytic structure of the present embodiment is significantly thicker. Conventionally, it has been difficult to support such a large amount of solid catalyst without interfering with its reaction activity, but in this embodiment, this problem is solved mainly by the use of a binder described below. The average thickness of the catalyst layer may be about 12 μm-180 μm, preferably about 15 μm-150 μm, and more preferably about 30 μm-100 μm.

Also, the thickness of the catalyst layer formed in the through-holes of the substrate is usually not necessarily uniform, depending on the shape of the through-holes. However, by forming a uniform catalyst layer on a plate substrate by the doctor blade method, or the like, and obtaining a correlation between the amount of the solid catalyst in the catalyst layer and the thickness thereof, the average thickness of the catalyst layer of the substrate relative to the weight of the supported solid catalyst, can be determined as the apparent thickness, even when through-holes are formed.

The average particle size of the particulate catalyst is not particularly limited, and may be, for example, 0.001 μm or more but 100 μm or less, preferably 0.005 μm or more but 100 μm or less, more preferably 0.01 μm or more but 80 μm or less, still more preferably 0.1 μm or more but 60 μm or less, particularly preferably 1.0 μm or more but 30 μm or less, and even more preferably 3.0 μm or more but 15 μm or less. This improves the efficiency of the catalytic reaction by providing a relatively large specific surface area of the catalyst and also prevents the catalyst from desorbing from the binder.

The term "average particle size" as used herein refers to the particle size at 50% in the volume-based particle size distribution (D50) as determined by the wet laser diffraction/scattering method. If measurement by the laser diffraction/-scattering method is difficult due to poor dispersion or the like, techniques such as scanning electron microscope observation, calculation from X-ray diffraction measurement, and imaging method can be applied.

The catalyst layer contains, along with the solid catalyst described above, a binder for immobilizing the solid catalyst. The binder contains at least cerium oxide. Cerium oxide contained in the binder does not interfere with the activity of the particulate catalyst and can make the carbonate ester generation reaction more efficient. The catalyst layer, which uses a binder consisting mainly of cerium oxide that is also used in the catalyst, can also suppress side reactions that may occur alongside the carbonate ester generation reaction.

The catalyst layer containing the binder can support a relatively large amount of catalyst. The binder enables the solid catalyst containing cerium oxide to be firmly immobilized on the substrate, and prevents desorption or exfoliation of the solid catalyst during the reaction. A catalytic structure having a catalyst layer containing such a binder can be used for a long period of time while maintaining high activity. The use of an inorganic binder also makes it possible to maintain high adhesion between the catalyst layer and the substrate without altering the property of the catalyst layer, even when the reaction temperature or regeneration treatment temperature is relatively high.

The binder may contain a component other than cerium oxide, such as a silica. The type of silica is not particularly limited and can be a silica formed using any compound as a precursor. These silicas may also be amorphous or crystalline. In addition, the binder may contain a tiny amount of alumina, magnesium, calcium, and impurities that may be introduced during the binder production step. Accordingly, the binder is preferably free of organic compounds and can be considered an inorganic binder.

The proportion of cerium oxide contained in the binder is preferably 1.0-10 mass%, more preferably 2.0-8.0 mass% or 3.0-7.0 mass%, and still more preferably 3.0-5.0 mass%, relative to the total weight of the catalyst layer. If the amount of cerium oxide as the binder contained in the catalyst layer is too large, pressure drop may increase, and if the amount is too small, binder capacity may be insufficient.

The proportion of cerium oxide in the binder is preferably 20 mass% or more or 30 mass% or more, more preferably 50 mass% or more or 70 mass% or more, and still more preferably 80 mass% or more or 90 mass% or more, relative to the total weight of the binder. More preferably, the binder consists essentially of cerium oxide, and particularly preferably consists only of cerium oxide.

Unlike cerium oxide in the form of catalyst particles, cerium oxide in the binder is present as a homogeneous component with no specific visible particles in the binder. Specifically, cerium oxide as a homogeneous component in the

binder is not present as fine particles, and is present in the binder in a state where cerium oxide is not present as particles even when observed under magnification. This is evident, for example, from the fact that cerium acetate completely dissolves in water, etc. and becomes a homogeneous component in the mixture of raw materials for the binder used in the step of producing the binder, which will be described in detail below.

The specific surface area of the particles of the solid component in the binder is not particularly limited, and may be, for example, 1 $m^2$/g or more but 1,000 $m^2$/g or less, and preferably 10 $m^2$/g or more but 500 $m^2$/g or less. The specific surface area of the binder in the above range allows an adequate diffusion rate of carbon dioxide and a monohydric alcohol in the binder, thereby accelerating the reaction on the surface of the solid catalyst and immobilizing the solid catalyst more firmly on the catalyst layer. The specific surface area can be measured by the BET method.

The amount of the supported solid component in the binder of the catalyst layer is, for example, 1 g/$m^2$ or more but 100 g/$m^2$ or less, and more preferably 1 g/$m^2$ or more but 40 g/$m^2$ or less. This allows for a larger exposed area of the solid catalyst, thereby improving the reaction efficiency and firmly immobilizing the solid catalyst on the substrate.

The solid component in the binder is contained in the catalyst layer at a concentration of, for example, 0.01 g or more but 5 g or less, and preferably 0.10 g or more but 1.0 g or less, per gram of solid catalyst. This allows for a larger exposed area of the solid catalyst, thereby improving the reaction efficiency and firmly immobilizing the solid catalyst on the substrate.

The solid component in the binder refers to inorganic oxide particles intentionally introduced into the binder and to a solidified substance of the binder. The mass proportion of the solid component in the inorganic binder is determined by dividing the mass of the residue left after drying and curing the inorganic binder alone by the mass of the inorganic binder before drying and curing. Therefore, the mass of the solid component in the inorganic binder can be calculated as (mass proportion of solid component) × (mass of inorganic binder applied).

(1.3. Intermediate layer)

An intermediate layer may be provided between the substrate and the catalyst layer. The intermediate layer contains, for example, a component that forms the binder described above, such as cerium oxide. An intermediate layer consisting mainly of or consisting of an inorganic binder is formed between the catalyst layer containing the solid catalyst and the substrate. By doing so, the adhesion of the catalyst layer to the substrate is further improved and desorption of the solid catalyst from the catalytic structure is prevented. Such an intermediate layer can also be provided to ensure sufficient adhesion between the catalyst layer and the substrate, even when the substrate is made of a material that has inherently low adhesion to the catalyst layer, for example, a metal substrate.

However, it is not necessary to provide an intermediate layer to ensure prevention of an increased pressure drop, side reactions that may be caused by a component other than the catalyst, generation of an unintended by-product, and the like.

The binder contained in the catalyst layer and the binder forming the intermediate layer may be different or the same in their compositions.

The amount of the supported solid component in the binder of the intermediate layer is, for example, 1 g/$m^2$ or more but 100 g/$m^2$ or less, and more preferably 10 g/$m^2$ or more but 50 g/$m^2$ or less. Within the above range, the adhesion between the catalyst layer and the substrate can be further improved while preventing cohesive failure of the intermediate layer.

In the catalytic structure for producing a carbonate ester according to the above-described embodiment, a relatively large amount of solid catalyst is firmly immobilized on the catalyst layer by the binder containing cerium oxide. The binder does not inhibit the catalytic reaction by the solid catalyst. When a carbonate ester is produced using such a catalytic structure for producing a carbonate ester, the efficiency of the carbonate ester generation reaction is excellent. Moreover, the solid catalyst is firmly immobilized on the substrate by an inorganic binder or the like, which prevents its release from the catalytic structure for producing a carbonate ester and also prevents the solid catalyst from powdering.

Such a catalytic structure for producing a carbonate ester can achieve high reaction efficiency, even at a fast liquid flow rate required for industrial processes. Since the solid catalyst is prevented from desorbing and powdering, it can be used repeatedly and is highly durable, even in the harsh environments required for industrial processes.

The catalytic structure for producing a carbonate ester according to the present embodiment has been described above, but the present invention is not limited to this embodiment.

For example, a catalytic structure for producing a carbonate ester according to an embodiment that differs from the above-described embodiment may be provided with a catalyst layer on a substrate, without the use of an intermediate layer consisting mainly of a binder. Thus, even when the intermediate layer is omitted, the solid catalyst in the catalyst layer is sufficiently immobilized by the binder containing cerium oxide, etc., preventing its desorption from the catalytic structure for producing a carbonate ester and the resulting powdering.

The boundary between the catalyst layer and the intermediate layer can be observed with an optical microscope or a scanning electron microscopy, or by elemental analysis with an EDS (energy dispersive X-ray spectrometer), or the

like.

By identifying the abundance of the metal component (e.g., cerium) in the catalyst layer by elemental analysis, the amount of the supported solid catalyst in the catalyst layer can be calculated based on the area of the catalyst layer and the mass supported by the entire catalyst layer. Specifically, if the component of the solid catalyst is cerium oxide, the amount can be determined by the following formula (I).

$$\text{(Amount of supported solid catalyst)} = [\text{(mass of catalyst layer)} \times \text{(mass proportion of cerium in catalyst layer)} \times \text{((mass of cerium oxide)/(mass of cerium))}]/\text{(area of catalyst layer)} \quad \text{(I)}$$

This value is synonymous with the mass of the solid catalyst in the unit area of the substrate where the solid catalyst is present, and can be determined with or without the intermediate layer.

As the elemental analysis method for identifying the metal component, scanning high-frequency inductively coupled plasma (ICP) method can be used. The amount of the supported binder in the catalyst and intermediate layers can be similarly calculated by determining the proportion of the solid component in the binder used to form the catalyst and intermediate layers, based on the proportion of the metal component determined by the above-described elemental analysis. Here, the area of the catalyst layer refers to the area where the solid catalyst (catalyst layer) is applied (formed). For example, if the substrate has a honeycomb-like shape and its periphery is not coated with the catalyst layer, it refers to the surface area of the entire substrate, which has internal communication holes formed and the catalyst layer applied. Here, the same idea can be applied to determine the amount of the supported solid catalyst, even in the presence of an intermediate layer.

[2. Method for producing catalytic structure for producing carbonate ester]

Next, a method for producing a catalytic structure for producing a carbonate ester according to the present embodiment will be described.

First, a substrate and a catalyst layer forming mixture for forming a catalyst layer are prepared. The catalyst layer forming mixture can be obtained by dissolving or dispersing a solid catalyst, a cerium oxide precursor as a binder material, or the like in a suitable liquid medium, such as water or an alcohol.

Next, a catalyst layer is formed on the surface of the substrate. Specifically, the catalyst layer is generated by first applying the catalyst layer forming mixture to the surface of the substrate, which is then dried and cured as follows.

The catalyst layer forming mixture can be applied by any method. For example, application can be performed by coating with a bar coater, a doctor blade, a roll coater, or a comma coater, coating by die coating, gravure coating, spin coating, or slit coating, inkjet, spraying, or immersion. In particular, when the substrate has through-holes, immersion, specifically by immersing the substrate in the catalyst layer forming mixture, is suitable. In order to increase the thickness of the catalyst layer, the application of the catalyst layer forming mixture may be repeated multiple times.

If the adhesion between the substrate and the catalyst layer is predicted to be insufficient, such as when a metal is used as the substrate, the following pretreatment step is preferably performed before the catalyst layer is formed. In the pretreatment step, for example, the substrate is washed with a volatile organic solvent such as alcohol or acetone to remove oil from the surface of the substrate, dried, and then the substrate is immersed in an aqueous alkaline solution, rinsed with water, and dried. Subsequently, the substrate is immersed in an aqueous acidic solution, rinsed with water, and dried. Alternatively, the surface of the substrate may be subjected to a chemical treatment, a heat treatment, a plasma treatment, a UV treatment, or a corona treatment.

The drying temperature of the catalyst layer forming mixture applied to the surface of the substrate can be any temperature at which the liquid medium can be removed, and it can be set according to the boiling point of the medium, etc. For example, it is 60°C or higher but 200°C or lower, preferably 70°C or higher but 160°C or lower, and more preferably 80°C or higher but 120°C or lower. The drying time is also suitably adjusted according to the desired dried conditions, or the like.

While the type of the cerium oxide precursor contained in the catalyst layer forming mixture is not particularly limited as long as it is a raw material that becomes cerium oxide after the calcination step described below, specific examples of the cerium oxide precursor include the following compounds. Examples include halides such as cerium chloride; inorganic salts such as cerium(III) nitrate (cerium nitrate) and cerium sulfate; carboxylates such as cerium acetate and cerium(III) 2-ethylhexanoate; cerium hydroxide; and complex compounds in which cerium is coordinated with a ligand such as acetylacetone, alkoxide (methoxide, ethoxide, tert-butoxide, etc.) (e.g., cerium(III) triacetylacetonate). Among these cerium oxide precursors, cerium sulfate, cerium nitrate, and cerium acetate are preferred, and cerium acetate is particularly preferred, from the viewpoint of their excellent adhesive action as a binder to immobilize catalyst particles after calcination.

Thus, a binder derived from a cerium oxide precursor, such as cerium acetate, can be used to suppress side

reactions caused by components other than the solid catalyst, such as alumina, and make the main reaction sufficiently efficient.

To produce cerium oxide from the above-described catalyst layer forming mixture, the mixture is calcined after application to the substrate. Specifically, the step of generating a binder contained in the catalyst layer involves a step of calcining a cerium oxide precursor, etc. contained in the mixture.

Thus, the temperature of the calcination step for curing the catalyst layer containing the binder can be set suitably according to the component of the mixture, and the like. For example, it is 200°C or higher but 900°C or lower, preferably 300°C or higher but 800°C or lower, more preferably 400°C or higher but 700°C or lower, or 450°C or higher but 750°C or lower, and still more preferably 500°C or higher but 700°C or lower, or 550°C or higher but 750°C or lower.

The time of the calcination step can also be adjusted suitably. For example, it is 10 minutes or longer but 10 hours or shorter, preferably 30 minutes or longer but 7 hours or shorter, and more preferably 1 hour or longer but 5 hours or shorter.

In the calcination step, the mixture applied to the substrate may be dried before it is calcined. Drying is performed, for example, by blowing air, where the drying temperature is, for example, 40°C or higher but 200°C or lower, preferably 50°C or higher but 150°C or lower, and more preferably 60°C or higher but 120°C or lower, or 65°C or higher but 130°C or lower. The drying temperature is still more preferably 70°C or higher but 110°C or lower, or 80°C or higher but 120°C or lower.

The drying time of the mixture can also be adjusted suitably. For example, it is 1 minute or longer but 30 minutes or shorter, preferably 3 minutes or longer but 20 minutes or shorter, and more preferably 5 minutes or longer but 15 minutes or shorter.

The calcination reaction, which results in the formation of cerium oxide from a precursor, such as cerium acetate, is considered to proceed according to the following schematic formula (II).

Low-humidity type $Ce(CH_3COO)_3 \rightarrow$ high-humidity type $Ce(CH_3COO)_3 \rightarrow Ce_8 \cdot O_3(CH_3COO)_{18} \rightarrow CeOCH_3COO \rightarrow Ce_2O_2CO_3 \rightarrow CeO_2$ ... (II)

Thus, cerium oxide is produced by thermal decomposition of the precursor upon calcination, which is the main process in the step of generating a binder. Unlike cerium oxide in the form of a particulate catalyst, cerium oxide in the binder obtained by the calcination step does not have a definite shape, and is present as a single homogeneous component in the binder. Cerium oxide in the form of a homogeneous component is contained in the binder, filling the gaps between the cerium particles of the catalyst.

In the case where the catalytic structure is provided with an intermediate layer, for example, the following additional steps may be required. In addition to the above-mentioned substrate and catalyst layer forming mixture, a binder stock solution for forming the intermediate layer is prepared. The binder stock solution can be obtained by dissolving or dispersing a binder material, such as a cerium oxide precursor, in a suitable liquid medium, such as water or an alcohol. The concentration of the binder material in the binder stock solution is not particularly limited and can be set suitably according to the method of applying the binder stock solution to the substrate.

Moreover, an intermediate layer is formed on the substrate before forming the catalyst layer. Specifically, the intermediate layer is obtained by applying, drying, and curing the above-mentioned binder stock solution on the substrate. The binder stock solution can be applied by any method. For example, application can be performed by coating with a bar coater, a doctor blade, a roll coater, or a comma coater, coating by die coating, gravure coating, spin coating, or slit coating, inkjet, spraying, or immersion. In particular, when the substrate has through-holes, immersion, specifically by immersing the substrate in the binder stock solution, is suitable. Note that, in order to increase the thickness of the intermediate layer, the application of the binder stock solution may be repeated multiple times.

Subsequently, the binder stock solution applied to the substrate is dried and cured to form an intermediate layer. The conditions for drying may be the same as the drying conditions for the catalyst layer forming mixture on the substrate described above. If the adhesion between the substrate and the intermediate layer is predicted to be insufficient, such as when a metal is used as the substrate, a pretreatment step similar to the pretreatment step performed prior to the formation of the catalyst layer described above may be performed before the intermediate layer is formed.

[3. Method for producing carbonate ester]

Next, a method for producing a carbonate ester will be described based on a preferred embodiment. The method for producing a carbonate ester according to this embodiment includes a step of reacting a monohydric alcohol and carbon dioxide in the presence of the above-described catalytic structure to produce a carbonate ester.

Before describing the method for producing a carbonate ester according to this embodiment, the mechanism of the reaction that occurs in this method will be described. First, the catalytic layer included in the catalytic structure catalyzes the reaction between a monohydric alcohol and carbon dioxide, represented by the following formula (1).

$$2ROH + CO_2 \leftrightarrow (RO)_2CO + H_2O \ ... \quad (1)$$

Here, the catalytic mechanism of the solid catalyst of the catalytic structure is considered to be a mechanism in which the alcohol dissociates and adsorbs on a basic site in the form of R-O-M (where M is the solid catalyst), forming RO-C(=O)-O...M with $CO_2$, while the alcohol adsorbs on an acidic site in the form of HO-R...M, generating RO-C(=O)-OR between both adsorbed species.

The reaction represented by formula (1) above is a reversible reaction. Therefore, if by-product water is present, it reacts with the generated carbonate ester again and returns to a monohydric alcohol and carbon dioxide. For this reason, it is preferable to perform the hydration reaction represented by formula (2) below using a hydration agent to remove the by-product water.

$$H_2O + R'CN \rightarrow R'C(=O)\text{-}NH_2 \ ... \quad (2)$$

As the hydration agent, a cyano group-containing compound represented by formula (2) above, or the like is used as described below.

By removing water through the reaction represented by formula (2) above, the reverse reaction in formula (1) is suppressed and the reaction to generate a carbonate ester is accelerated. Although the reason is not clear, the solid catalyst is also considered to be catalytically active for the hydration reaction represented by formula (2) above. Heretofore, the mechanism of the reaction that occurs in this method has been described.

As described above, the method for producing a carbonate ester according to this embodiment includes a step of reacting a monohydric alcohol and carbon dioxide in the presence of the above-described catalytic structure and hydration agent to produce a carbonate ester.

Specifically, the reaction for a carbonate ester is carried out by installing the catalytic structure in a reaction vessel and introducing a hydration agent, a monohydric alcohol, and carbon dioxide into the reaction vessel.

As the monohydric alcohol, one or more compounds selected from primary alcohols, secondary alcohols, and tertiary alcohols can be used.

Specifically, examples of the monohydric alcohol include methyl alcohol, ethyl alcohol, n-propyl alcohol, isopropyl alcohol, n-butyl alcohol, isobutyl alcohol, sec-butyl alcohol, tert-butyl alcohol, n-pentyl alcohol, n-hexanol, 2-hexanol, 3-hexanol, heptanol, hexanol, and phenol.

The type of the monohydric alcohol can be suitably selected according to the chemical structure of the desired carbonate ester and the method for using the resulting carbonate ester.

While the hydration agent, which is an optional component, is not particularly limited as long as it can react with water to remove water, examples thereof include cyano group-containing compounds represented by formula (2) above, where one of them can be used alone or two or more of them can be used in combination. Specifically, examples include acetonitrile, cyanoethane, 1-cyanopropane, 2-cyanopropane, cyanoethylene, phenylacetonitrile, benzonitrile, 2-cyano-pyridine, 2-cyanopyrazine, 2-cyanopyrimidine, thiophene-2-carbonitrile, and 2-furonitrile (2-cyanofuran). In particular, 2-cyanopyridine is preferred in terms of water removal efficiency, i.e., hydration reaction rate.

The amount of the hydration agent used can be determined according to the amount of the monohydric alcohol used. For example, the amount of the hydration agent is 0.01 moles or more but 2 moles or less, and preferably 0.1 moles or more but 0.5 moles or less, per mole of the monohydric alcohol used.

While the temperature of the reaction for generating a carbonate ester is not particularly limited, it is preferably 50°C or higher but 300°C or lower. If the reaction temperature is lower than 50°C, the reaction rate may be low depending on the type of the reaction substrate, and both the carbonate ester synthesis reaction and the hydration reaction with the hydration agent may hardly proceed, resulting in low productivity of the carbonate ester. On the other hand, if the reaction temperature exceeds 300°C, the reaction rate of each reaction increases, but depending on the type of the reaction substrate, the monomer of the carbonate ester or the amide obtained by the hydration reaction may easily denature into another monomer or polymerize, resulting in a low yield of the carbonate ester. The reaction temperature is more preferably 100°C or higher but 200°C or lower. However, since this reaction temperature is considered to vary depending on the type and amount of the solid catalyst and the amount and ratio of the raw materials (monohydric alcohol, hydration agent), the optimal conditions are preferably determined accordingly.

While the reaction pressure is not particularly limited, it is preferably 0.1 Mpa or higher but 20 Mpa or lower (absolute pressure). If the reaction pressure is lower than 0.1 MPa (absolute pressure), a decompression device will be required, which not only complicates the equipment and increases the cost, but also requires power energy for decreasing the pressure, resulting in a tendency to render the energy efficiency poor. On the other hand, if the reaction pressure exceeds 20 MPa, the hydration reaction with the hydration agent may be difficult to proceed depending on the type of the hydration agent, which not only lowers the yield of the carbonate ester, but also requires power energy for increasing the pressure, thereby rendering the energy efficiency poor. From the viewpoint of increasing the yield of the carbonate ester,

the reaction pressure is more preferably 0.1 MPa or higher but 10 MPa or lower (absolute pressure).

While the reaction time is not particularly limited and can be determined suitably according to the type of the reaction substrate (raw materials), the type of the hydrate, and the generation rate of the by-products, it is, for example, 5 minutes or longer but 24 hours or shorter, preferably 15 minutes or longer but 8 hours or shorter, and more preferably 30 minutes or longer but 6 hours or shorter. When a continuous reactor is used, the reaction time (residence time) can be defined as the total time from introducing the raw materials into the reaction vessel to discharging the resulting product from the reaction vessel, taking into account the flow rate of the raw materials. When the reaction is carried out by circulating the reaction substrate through the catalytic structure, the circulating flow rate is defined by the space velocity expressed by the following formula (3).

$$\text{Space velocity (per minute)} = \text{circulating flow rate } (m^3/\text{min}) \div \text{volume of catalytic structure } (m^3) \qquad (3)$$

While the value of the space velocity is not particularly limited, it is, for example, 0.005 or more but 5,000 or less per minute, preferably 0.05 or more but 500 or less per minute, and more preferably 0.5 or more but 50 or less per minute. If the space velocity is too small, $CO_2$ will be consumed before it passes through the catalytic structure, which will render the reaction efficiency poor and may increase side reactions. On the other hand, a greater space velocity results in a larger pump size, which may render the energy efficiency poor. A carbonate ester can be produced efficiently by a step of circulating a reaction substrate containing the raw materials through a carbonate ester production device at a space velocity (per minute) in the range mentioned above, for example, 0.005 or more but 5,000 or less.

Thus, a carbonate ester can be produced efficiently. In addition, because the catalytic structure according to the above-described embodiment is used in the present embodiment, the solid catalyst is prevented from powdering and desorbing from the catalytic structure. Therefore, the catalytic structure can be used repeatedly even in the harsh environments required for industrial processes. In addition, good activity of the solid catalyst can be maintained over a longer period of time by adjusting the catalyst component.

[4. Carbonate ester production device]

Next, a carbonate ester production device will be described based on a preferred embodiment. The carbonate ester production device comprises the above-mentioned catalytic structure for producing a carbonate ester. For example, the carbonate ester production device has a housing (casing). The housing is made of a SUS pipe, or the like, and accommodates a catalytic structure for producing a carbonate ester inside. The carbonate ester production device preferably has multiple catalytic structures for producing a carbonate ester, where these catalytic structures for producing a carbonate ester are arranged, for example, in series.

The carbonate ester production device is preferably equipped with feed channels for feeding a monohydric alcohol and carbon dioxide, i.e., the raw materials for producing a carbonate ester, to the catalytic structure for producing a carbonate ester.

When the aforementioned raw materials are fed to the carbonate ester production device via such feed channels, carbonate ester generation reaction proceeds inside the catalytic structure for producing a carbonate ester. The carbonate ester resulting from the carbonate ester generation reaction is preferably discharged, together with unreacted raw materials, etc., from the carbonate ester production device via a discharge channel.

In order to adjust the temperature of the carbonate ester generation reaction, a heat transfer fluid is preferably fed to the carbonate ester production device. The heat transfer fluid allows for an increase in the temperature of the catalytic structure for producing a carbonate ester and for adjustment of the reaction temperature. For this purpose, the carbonate ester production device is preferably equipped with a heat transfer fluid feed pipe for feeding the heat transfer fluid, and the heat transfer fluid fed to the carbonate ester production device from the inlet of the heat transfer fluid feed pipe is discharged outside the carbonate ester production device, for example, via an outlet. Thereafter, the heat transfer fluid is, for example, adjusted to a predetermined temperature and then fed again into the carbonate ester production device via the inlet of the heat transfer fluid feed pipe.

The form of the carbonate ester production device is not limited to that described above. For example, the carbonate ester production device may be designed to have a shape that allows for large-scale implementation, which increases the flow rate of the fluid containing the raw materials that are fed through the aforementioned feed channel. In the case where the size of the carbonate ester production device is increased as described above, the integrated structures, each consisting of the catalytic structures for producing a carbonate ester arranged in parallel, may be arranged in series in the height direction. In the carbonate ester production device having such a structure, only the integrated structure including a potentially deteriorated catalytic structure can be easily replaced after long-term operation, thus minimizing the device operating costs for the catalysis. It is preferable to apply a sealing structure to the integrated structure, which has the catalytic structures for producing a carbonate ester arranged in parallel, to ensure that the fluid can always pass through

the catalytic structures. When the integrated structures, each consisting of the catalytic structures for producing a carbonate ester arranged in parallel, are arranged in series in the height direction, it is preferable to provide space between each stage to ensure uniform flow of the fluid in the carbonate ester production device. In order to minimize the size of the device by densely arranging the catalytic structures along the cross-section of the carbonate ester production device, for example, rectangular-shaped catalytic structures may be used. The rectangular-shaped catalytic structures allows multiple catalytic structures to be densely arranged along the cross-section of the carbonate ester production device, thus minimizing the loss of storage space. However, the shape of the catalytic structure is also not particularly limited, and the catalytic structure may have a shape other than a rectangular shape. The catalytic structure preferably has a porous, foam-like, honeycomb-like, or mesh-like structure.

The integrated structure preferably includes a securing plate that is a part of the housing and that supports the multiple catalytic structures. Such a securing plate is formed, for example, with frames corresponding to the outline of the catalytic structures. The catalytic structures are preferably fitted into the frame and fixed in place in a removable manner.

Alternatively, instead of such a catalytic structure, the integrated structure may include a rectangular honeycomb-like structure that does not support a catalyst. This structure, which has a rectangular honeycomb structure but does not support a catalyst, can be employed to prevent the total amount of the catalyst contained in the carbonate ester production device from becoming excessive. An integrated structure including several tens of catalytic structures, for example, 30-40 catalytic structures, can be equipped with a few (e.g., one or two) structures that do not support a catalyst so as to adjust the amount of catalyst distribution to an appropriate level and suppress the amount of by-products generated.

A carbonate ester production device, which has a structure in which catalytic structures are arranged not only in series but also in parallel as described above, i.e., a structure in which a plurality of integrated structures, each consisting of multiple catalytic structures arranged in parallel, are arranged in series, is suitable for use in a reaction system in which the flow rate of the fluid containing the raw materials is, for example, 500 $m^3$/h or more, preferably 550 $m^3$/h or more. If a fluid is fed at a flow rate smaller than these values, for example, a carbonate ester production device, which has ten or less catalytic structures arranged only in series, is used.

[5. Carbonate ester production facility]

Next, a carbonate ester production facility, which is equipped with the above-described carbonate ester production device, and the like, will be described based on a preferred embodiment. As illustrated in Figure 1, a carbonate ester production facility 100 comprises, for example, a carbonate ester production device 60, raw material feed systems for feeding raw materials to the carbonate ester production device 60, and a recovery system for collecting the generated carbonate ester.

When the internal pressure of a $CO_2$ storage facility 72, as the feed system for a raw material, is increased by a $CO_2$ booster pump 73, $CO_2$ in the $CO_2$ storage facility 72 is fed to a buffer tank 74. Meanwhile, a liquid monohydric alcohol and a hydration agent such as 2-cyanopyridine, which are raw materials stored in a raw material tank 76, are fed from the raw material tank 76 to the buffer tank 74 by a raw material feed pump 78. Thus, carbon dioxide, the monohydric alcohol, and the like fed to the buffer tank 74 through separate systems are mixed in the buffer tank 74. The mixture is fed to the carbonate ester production device 60 via a first filter 80.

In the carbonate ester production device 60, a catalytic structure for producing a carbonate ester (not shown) accelerates the carbonate ester generation reaction to generate a carbonate ester according to the type of the monohydric alcohol used as the raw material. The resulting carbonate ester is fed to the recovery system via a second filter 82. In the recovery system, the liquid containing the carbonate ester is separated from the liquid containing the unreacted raw materials, i.e., the monohydric alcohol and the hydration agent such as 2-cyanopyridine. Under the action of a reaction solution circulation pump 84, the liquid containing the carbonate ester is then sent to a liquid extraction pump 86, while the liquid containing the monohydric alcohol, etc., is returned to the buffer tank 74. In the liquid extraction pump 86, liquid other than the carbonate ester is removed to produce a high-purity carbonate ester.

EXAMPLES

Hereinafter, the present invention will be described in more detail by way of examples and comparative examples, although the present invention is not particularly limited to these examples and comparative examples.

<Production example 1: production example of catalytic structure made of cerium oxide>

700 g of cerium oxide (manufactured by Solvay Special Chem Japan, Ltd., HSA-20SP, $CeO_2$ powder, average particle size: about 10 μm) as a catalyst, 70 g of cerium acetate hydrate ($Ce(CH_3CO_2)_3 \cdot 1H_2O$) as a raw material of a binder, and 700 g of ion-exchanged water were placed in a ball mill and the mixture was ground for 30 minutes. To the resulting ground substance, 500 g of ion-exchanged water was added to obtain slurry (s).

A cordierite honeycomb (ceramic honeycomb) was immersed in the above slurry (s). After confirming that the entire honeycomb had been immersed, it was pulled out, blown with air, dried at 100°C, and then calcined at 600°C to give a catalytic structure (a).

The ceramic honeycomb used here was a cordierite honeycomb, ceramic catalyst support "HONEYCERAM" (registered trademark) manufactured by NGK Insulators, Ltd. The purchased product, which had a cell density of 400 cpsi (400 cells/inch$^2$ = 62 cells/cm$^2$) and a wall thickness of 4.5 mil core (114 μm = 0.114 mm), was cut out using a punch drill and cutting machine to obtain a ceramic honeycomb with a size of 40 mm diameter x 50 mm length.

The aforementioned catalytic structure (a) was immersed again in the aforementioned slurry (s). After confirming that the entire catalytic structure had been immersed, it was pulled out, blown with air, dried at 100°C, and then calcined at 600°C to give a catalytic structure (b1).

The resulting catalytic structure (b1) supported 5.1 g/honeycomb structure, i.e., 81 g/L (mass supported per volume) or 28 g/m$^2$ (mass supported per geometric surface area (per unit area of the catalyst layer)), of particulate cerium oxide as a catalyst.

The binder contained in the catalytic structure (b1) was formed solely from cerium oxide ($CeO_2$) derived from cerium acetate hydrate ($Ce(CH_3CO_2)_3 \cdot 1H_2O$) as a raw material.

By the same production process as the catalytic structure (b1), a catalytic structure (b2), which supported 5.4 g/honeycomb structure, i.e., 85 g/L (mass supported per volume) or 30 g/m$^2$ (mass supported per geometric surface area), of particulate cerium oxide as a catalyst, was also obtained.

<Production example 2>

A catalytic structure (c) was obtained in the same manner as in Example 1, except that the cell density of the ceramic honeycomb was changed to 600 cpsi (600 cells/inch$^2$ = 93 cells/cm$^2$).

The resulting catalytic structure (c) supported 5.0 g/honeycomb structure, i.e., 80 g/L (mass supported per volume) or 27 g/m$^2$ (mass supported per geometric surface area), of particulate cerium oxide as a catalyst.

<Production example 3>

The aforementioned catalytic structure (c) was immersed again in the aforementioned slurry (s). After confirming that the entire catalytic structure had been immersed, it was pulled out, blown with air, dried at 100°C, and then calcined at 600°C to give a catalytic structure (d).

The resulting catalytic structure (d) supported 8.6 g/honeycomb structure, i.e., 137 g/L (mass supported per volume) or 39 g/m$^2$ (mass supported per geometric surface area), of particulate cerium oxide as a catalyst.

<Production example 4>

The aforementioned catalytic structure (d) was immersed again in the aforementioned slurry (s). After confirming that the entire catalytic structure had been immersed, it was pulled out, blown with air, dried at 100°C, and then calcined at 600°C to give a catalytic structure (d).

The resulting catalytic structure (e) supported 15.6 g/honeycomb structure, i.e., 248 g/L (mass supported per volume) or 71 g/m$^2$ (mass supported per geometric surface area), of particulate cerium oxide as a catalyst.

<Comparative production example 1: production example of catalytic structure made of alumina>

700 g of cerium oxide (manufactured by Solvay Special Chem Japan, Ltd., HSA-20SP, $CeO_2$ powder) as a catalyst, 21 g of alumina powder as a raw material of a binder, and 700 g of ion-exchanged water were placed in a ball mill and the mixture was ground for 30 minutes to give slurry (n).

A cordierite honeycomb (ceramic honeycomb) was immersed in the above slurry (n). After confirming that the entire honeycomb had been immersed, it was pulled out, blown with air, dried at 100°C, and then calcined at 600°C to give a catalytic structure (e).

The ceramic honeycomb used here was the same as that used in Production example 1.

The aforementioned catalytic structure (e) was immersed again in the aforementioned slurry (n). After confirming that the entire catalytic structure had been immersed, it was pulled out, blown with air, dried at 100°C, and then calcined at 600°C to give a catalytic structure (f1).

The resulting catalytic structure (f1) supported 4.2 g/honeycomb structure, i.e., 67 g/L (mass supported per volume) or 23 g/m$^2$ (mass supported per geometric surface area), of particulate cerium oxide as a catalyst.

<Comparative production example 2>

The aforementioned catalytic structure (f1) was immersed again in the aforementioned slurry (n). After confirming that the entire catalytic structure had been immersed, it was pulled out, blown with air, dried at 100°C, and then calcined at 600°C to give a catalytic structure (f2).

The resulting catalytic structure (f2) supported 8.3 g/honeycomb structure, i.e., 132 g/L (mass supported per volume) or 46 g/$m^2$ (mass supported per geometric surface area), of particulate cerium oxide as a catalyst.

<Example 1>

The catalytic structures (b1) and (b2) obtained in Production example 1 were used to produce a carbonate ester under the following conditions. The results are shown in Table 1.

- Equipment: Continuous flow reactor (see Figure 1)
- Feed mole ratio: (1-propanol (PrOH))/(2-cyanopyridine (2-CP))/(catalyst ($CeO_2$)) = 600/100/1
- Circulation flow rate: 720 mL/minute
- Residence time: 4 h
- Reaction pressure: 2.0 MPa
- Reaction temperature: 132°C
- Catalytic structure: Total of 2 catalytic structures were used (total amount of catalyst supported; 10.5 g)

<Examples 2-6, and Comparative examples 1 and 2>

Carbonate esters were produced in the same manner as in Example 1, except that the reaction conditions were changed as shown in Table 1. The results are shown in Table 1.

[Table 1]

| | | Example 1 | | Example 2 | | Example 3 | | Example 4 | Example 5 | Example 6 | Comparative example 1 | | Comparative example 2 |
|---|---|---|---|---|---|---|---|---|---|---|---|---|---|
| Catalytic structure | Type of catalytic structure | Production example 11 (b1) and (b2) | | Production example 1/ (b1) and (b2) | | Production example 2/(c) | | Production example 3/(d) | Production example 3/(d) | Production example 4/(e) | Comparative production example 1/(f1) | | Comparative production example 2/(f2) |
| | Catalytic particles | Cerium oxide | | Cerium oxide | | Cerium oxide | | Cerium oxide | Cerium oxide | Cerium oxide | Cerium oxide | | Cerium oxide |
| | Component of binder | Cerium oxide (derived from cerium acetate) | | Cerium oxide (derived from cerium acetate) | | Cerium oxide (derived from cerium acetate) | | Cerium oxide (derived from cerium acetate) | Cerium oxide (derived from cerium acetate) | Cerium oxide (derived from cerium acetate) | Alumina | | Alumina |
| | Material of substrate | Ceramic | | Ceramic | | Ceramic | | Ceramic | Ceramic | Ceramic | Ceramic | | Ceramic I |
| | ture of substrate/cell d | Honeycomb/ 400 cpsi | | Honeycomb/ 400 cpsi | | Honeycomb/ 600 cpsi | | Honeycomb/ 600 cpsi | Honeycomb/ 600 cpsi | Honeycomb/ 600 cpsi | Honeycomb/ 400 cpsi | | Honeycomb/ 400 cpsi |
| Amount of catalyst supported | (g/honeycomb structure) | 5.1 | 5.4 | 5.1 | 5.4 | 5.0 | 5.0 | 8.6 | 8.6 | 15.6 | 4.2 | 4.2 | 8.3 |
| | Mass supported per volume (g/L) | 81 | 85 | 81 | 85 | 80 | 80 | 137 | 137 | 248 | 67 | 67 | 132 |
| | Mass supported per geometric surface area ($g/m^2$) | 28 | 30 | 28 | 30 | 27 | 27 | 39 | 39 | 71 | 23 | 23 | 46 |
| | Total mass of catalyst supported (g) | 10.5 g (two) | | 10.5 g (two) | | 10.0 g (two) | | 8.6 g (one) | 8.6 g (one) | 15.6 g (one) | 8.4 g (two) | | 8.3 g (one) |

(continued)

| | | Example 1 | Example 2 | Example 3 | Example 4 | Example 5 | Example 6 | Comparative example 1 | Comparative example 2 |
|---|---|---|---|---|---|---|---|---|---|
| Conditions for carbonate ester generation reaction | Monohydric alcohol | 1-Propanol | 1-Propanol | 1-Propanol | 1-Propanol | 1-Propanol | 1-Propanol | 1-Propanol | 1-Propanol |
| | Hydration agent | 2-Cyanopyridine | 2-Cyanopyridine | 2-Cyanopyridine | 2-Cyanopyridine | 2-Cyanopyridine | 2-Cyanopyridine | 2-Cyanopyridine | 2-Cyanopyridine |
| | $CeO_2$/ 2-cyanopyridine/ 1-propanol (mole ratio) | 1/100/600 | 1/100/600 | 1/100/600 | 1/100/600 | 1/60/360 | 11601360 | 1/100/600 | 1/100/600 |
| | Reaction pressure (MPa) | 2.0 | 0.8 | 0.8 | 0.8 | 0.8 | 0.8 | 2.0 | 2.0 |
| | Yield of carbonate ester after 7 hours of reaction (mol%) | 43.2 | 35.2 | 36.6 | 31.4 | 44.9 | 48.1 | 6.2 | 2.6 |
| | By-product/carbonate ester after 7 hours of reaction (mol%) | 0.74 | 1.39 | 1.07 | **1.01** | 1.60 | 2.20 | 0.82 | 2.10 |

As can be appreciated from the results in Table 1, in the examples where cerium oxide derived from cerium acetate was used as a binder, the yield of the carbonate ester, i.e., the desired compound, was high and the side reactions were suppressed. In these examples, the use of cerium oxide, which was used in both the catalyst component and the binder, was believed to have prevented side reactions, allowing only the main reaction by the catalyst to proceed.

In contrast, the comparative examples using alumina as a binder showed a very low yield of the desired carbonate ester and generated more by-products than the examples. In the comparative examples, alumina contained in the binder may have also acted as a secondary catalyst, causing unintended side reactions.

Although preferred embodiments of the present invention have been described in detail above, the present invention is not limited to these specific examples. A person having ordinary skill in the art to which the present invention pertains would obviously conceive of various alterations and modifications within the scope of the technical idea stated in the claims, and such alterations and modifications should be construed as being duly within the technical scope of the present invention.

DESCRIPTION OF REFERENCE NUMERALS

60 Carbonate ester production device
100 Carbonate ester production facility

## Claims

1. A method for producing a carbonate ester, comprising:

   a step of reacting a monohydric alcohol and carbon dioxide in the presence of a catalytic structure to produce a carbonate ester,
   wherein the catalytic structure comprises a substrate, and a catalyst layer formed on at least a portion of the surface of the substrate and containing at least a solid catalyst and a binder,
   the solid catalyst comprises particulate cerium oxide, and
   the binder comprises cerium oxide as a homogeneous component.

2. The method for producing a carbonate ester according to claim 1, wherein the cerium oxide contained in the binder is derived from at least one of the following cerium compounds: cerium acetate, cerium sulfate, and cerium nitrate.

3. The method for producing a carbonate ester according to claim 2, further comprising a binder generating step for generating the binder,
   wherein, in the binder generating step, the cerium compound is calcined to obtain cerium oxide.

4. The method for producing a carbonate ester according to claim 2, wherein the content of the cerium oxide derived from the cerium compound in the catalyst layer is 1.0-10% by weight relative to the total weight of the catalyst layer.

5. The method for producing a carbonate ester according to claim 1, wherein the substrate is ceramic.

6. The method for producing a carbonate ester according to claim 1, wherein the mass of the supported solid catalyst in the catalyst layer is 15 $g/m^2$ or more but 200 $g/m^2$ or less.

7. The method for producing a carbonate ester according to claim 1, wherein a hydration agent is used to remove by-produced water in the carbonate ester generation reaction.

8. A catalytic structure for producing a carbonate ester, comprising:

   a substrate; and
   a catalyst layer formed on at least a portion of the surface of the substrate and containing at least a solid catalyst and a binder,
   wherein the solid catalyst comprises particulate cerium oxide, and
   the binder comprises cerium oxide as a homogeneous component.

**9.** The catalytic structure for producing a carbonate ester according to claim 8, wherein the content of the cerium oxide as a homogeneous component in the catalyst layer is 1.0-10% by weight relative to the total weight of the catalyst layer.

**10.** The catalytic structure for producing a carbonate ester according to claim 8, wherein the substrate is ceramic.

**11.** The catalytic structure for producing a carbonate ester according to claim 8, wherein the mass of the supported solid catalyst in the catalyst layer is 15 g/m$^2$ or more but 200 g/m$^2$ or less.

**12.** The catalytic structure for producing a carbonate ester according to claim 8, wherein the substrate has a honeycomb structure and the cell density of the substrate is 15-200 (cells/cm$^2$), or the wall thickness of the substrate is 0.01-2.0 mm.

Figure 1

100
80
72
60
73
P
G
74
P
G
82
84
76
78
86

## INTERNATIONAL SEARCH REPORT

International application No. **PCT/JP2024/038655**

**A. CLASSIFICATION OF SUBJECT MATTER**

***C07C 68/04***(2006.01)i; ***B01J 23/10***(2006.01)i; ***C07B 61/00***(2006.01)i; ***C07C 69/96***(2006.01)i
FI: C07C68/04 A; B01J23/10 Z ZAB; C07B61/00 300; C07C69/96 Z

According to International Patent Classification (IPC) or to both national classification and IPC

**B. FIELDS SEARCHED**

Minimum documentation searched (classification system followed by classification symbols)

B01J21/00-38/74, C07B31/00-63/04, C07C1/00-409/44

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2020/013135 A1 (NIPPON STEEL CORPORATION) 16 January 2020 (2020-01-16) | 1-12 |
| A | CN 107735161 A (BASF CORP.) 23 February 2018 (2018-02-23) | 1-12 |
| A | JP 06-211751 A (NIPPON SHOKUBAI CO., LTD.) 02 August 1994 (1994-08-02) | 1-12 |
| A | CN 116870892 A (UNIVERSITY OF SCIENCE AND TECHNOLOGY OF CHINA) 13 October 2023 (2023-10-13) | 1-12 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 January 2025** | **21 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/038655**

| Patent document cited in search report | Publication date (day/month/year) | Patent family member(s) | Publication date (day/month/year) |
|---|---|---|---|
| WO 2020/013135 A1 | 16 January 2020 | US 2021/0284594 A1 | |
| | | EP 3822251 A1 | |
| | | CN 112424155 A | |
| | | KR 10-2021-0030376 A | |
| | | SG 11202012740P A | |
| | | TW 202005947 A | |
| CN 107735161 A | 23 February 2018 | US 2018/0043335 A1 | |
| | | WO 2016/141140 A1 | |
| | | EP 3265212 A1 | |
| | | KR 10-2017-0128408 A | |
| | | BR 112017018826 A | |
| JP 06-211751 A | 02 August 1994 | US 5430170 A | |
| | | KR 10-1994-0011430 A | |
| | | TW 228519 B | |
| CN 116870892 A | 13 October 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012162523 A
- WO 2020013135 A